# EUROPEAN PATENT SPECIFICATION

(11) **EP 0 367 338 B1**
(45) Date of publication and mention of the grant of the patent: **11.08.1993**
(21) Application number: 89202704.6
(22) Date of filing: 25.10.1989
(51) Int. Cl.: A61N 1/08

(54) **Electrotherapy apparatus**
Elektrotherapie-Gerät
Appareil d'électrothérapie

(30) Priority: 31.10.1988 NL 8802670
(43) Date of publication of application: 09.05.1990
(73) Proprietor: INGENIEURSBURO UNIPHY B.V., NL-5652 AW Eindhoven (NL)
(72) Inventor: Beun, Nicolaas Herman, NL-5623 JM Eindhoven (NL); van den Heuvel, Hendrik Louis Maria, NL-5501 JB Eindhoven (NL)
(74) Representative: de Bruijn, Leendert C.

(56) References cited:
- EP-A- 0 026 324
- EP-A- 0 026 479
- EP-A- 0 269 845
- DE-B- 2 810 046
- FR-A- 2 507 900
- US-A- 3 791 373
- US-A- 4 600 010
- L'ONDE ELECTRIQUE, vol. 54, no. 8, 1974, pages 423-428; R.J. PLASZCZYNSKI: "Normes auxquelles doit répondre le matériel d'électronique médicale"

## Description

The invention relates to an electrotherapy apparatus which is capable of generating direct currents or alternating currents which can be applied to a patient via electrodes.

Such apparatuses are, for example, known from EP-0,054,654, EP-A-0,026,324 and DE-A-3,701,473. In particular, the last-mentioned publication describes a protective circuit with which a short circuit can be brought about between the electrodes with the aid of a photosensitive triac circuit in order to avoid undesirable high current peaks which may be painful or dangerous for a patient occurring in the output circuit on switching the apparatus on or off or in the event of mains supply failures. The triac circuit mentioned is controlled by a detector circuit which monitors the supply voltage of the apparatus.

The object of the invention is now to provide an electrotherapy apparatus of this type with a much more extensive protection so that, even during normal operation and regardless of any mains voltage variations, the current which is applied to the patient is monitored and if necessary, is reduced if the current should exceed a predetermined value.

Within the framework of this object, the invention now provides an electrotherapy apparatus which comprises:
- two voltage/current converters, each provided with a current input, a current output and a control voltage input, the current input of each of these converters being connected via a resistor to a first supply voltage terminal and the control voltage input being connected to a waveform generator,
- a transformer having a primary and a secondary winding, which secondary winding is connected via at least one relay contact to an output circuit which can be connected to the said electrodes, the two ends of the primary winding each being connected to a current output of one of the said converters and a centre tapping of the primary winding being connected to the second supply voltage terminal,
- there being connected to the two said resistors a protective circuit which monitors the current through said resistors during operation and which, if a predetermined current is exceeded, operates a relay, as a result of which the said at least one relay contact is switched over and the connection between the output circuit and the secondary winding of the transformer is interrupted.

In the apparatus according to the invention, the current delivered by each voltage/current converter to the primary winding of the transformer also flows through the resistor which is connected between the current input of the converter concerned and the first supply voltage terminal. As a result, therefore, of monitoring the current through this resistor, the current through the primary winding of the transformer is also monitored, as also is the currrent associated therewith through the secondary winding of the transformer to the electrodes.

In addition, due to the use of a transformer, a direct galvanic coupling between the suppply voltage terminal and of the apparatus and the patient is avoided. As a result of using a bridge rectifier connected to the secondary side of the transformer, it is possible to feed not only alternating currents, but also direct currents to a patient via the electrodes.

The current through the said resistors may be monitored in various ways. According to a first preferred embodiment, the protective circuit is provided with a first and a second comparator, each having an input connected to the junction between one of the said resistors and the current input connected thereto, and each having the other input connected, or connectable, to a reference voltage source in a manner such that, during operation, the voltage across each of the said resistors is compared in a comparator with a predetermined reference voltage and that, if the reference voltage is exceeded, the comparator concerned generates an energizing signal for the said relay. The reference voltage is preferably derived from adjustment means with which the user of the apparatus can set the desired output current. The first and second comparator then ensure that the voltage across the said resistors never exceeds, for example, 150% of the voltage across it if the set output current flows through said resistors.

In a further embodiment of the apparatus, the protective apparatus is provided with a third comparator, one input of which is connected via a resistor network to the said resistors and the other input of which is connected, or is connectable, to a limit voltage source in a manner such that, during operation, the sum of the voltages across the said resistors is compared in the third comparator with a predetermined limit value, the third comparator generating an energizing signal for the said relay if the limit value is exceeded. This third comparator therefore ensures that, regardless of the set reference value, the output current never exceeds a predetermined limit value which can be specified on the basis of safety regulations, approval requirements and the like and which can, for example, be permanently set by the manufacturer of the apparatus.

In general, it will be desirable that the said limit value is dependent on the instantaneous frequency. According to a preferred embodiment, therefore, the limit voltage source is provided with a frequency-dependent network such that the instantaneous limit value is dependent on the instantaneous frequency of the sum of the currents through the said resistors.

Not only the amplitude of the current pulses which are delivered to the transformer by the voltage/current converters has an effect on the total current level on the secondary side, but the shape of the current pulses may also have an effect. In particular, an increasing width of the current pulses is of importance in that case. In connection therewith, in a further preferred embodiment, the protective circuit is provided with a fourth comparator, one input of which is connected via an integrating network to the junction between one of the said resistors and the associated current input and the other input of which is connected to a standard voltage source in a manner such that, during operation, the integrated voltage across the said one resistor concerned is compared with the standard voltage and that, if the standard voltage is exceeded, an energizing signal is generated by the fourth comparator for the said relay.

The invention will be explained in more detail below with reference to the accompanying figures.

Figure 1 shows a block diagram of the electrotherapy apparatus according to the invention.

Figure 2 shows, in a block diagram, how the current/voltage converters in the apparatus in Figure 1 are supplied.

Figure 3 shows another embodiment of the appparatus according to the invention.

Figure 4 illustrates a detailed embodiment of the amplitude adjustment circuit in Figure 3.

Figure 5 shows a further embodiment of an apparatus according to the invention.

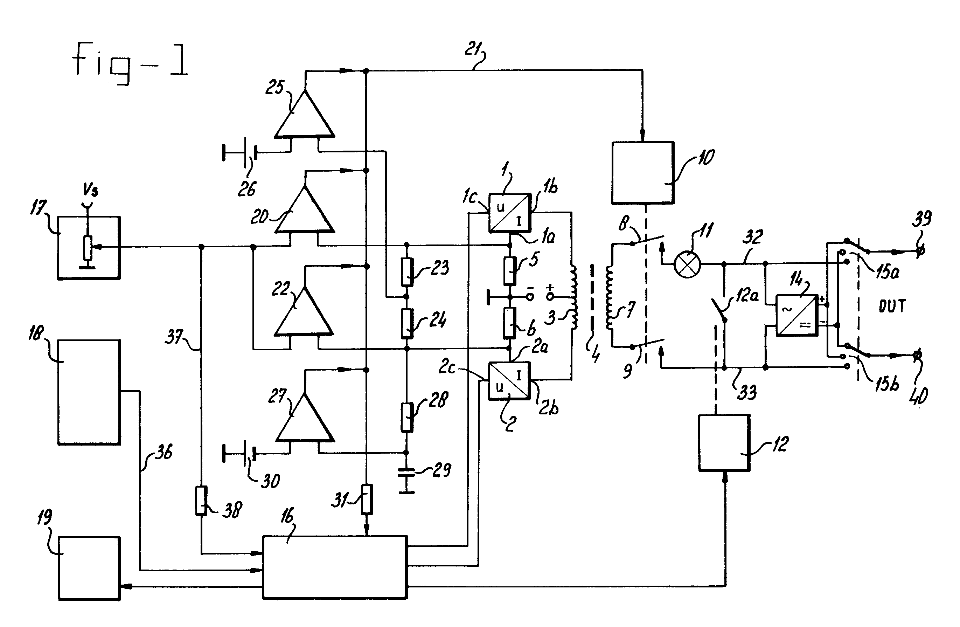

The electrotherapy apparatus shown diagrammatically in Figure 1 comprises two voltage/current converters 1 and 2, each provided with a current input 1a, 2a, a current output 1b, 2b, and a control input 1c, 2c. The current outputs 1b and 2b are each connected to an end of a primary winding 3 of an output transformer 4. This primary winding 3 is further provided with a centre tapping which is connected to a first (in this case the positive) supply voltage terminal. The current inputs 1a and 2a are each connected via a resistor 5 or 6 respectively to the second (in this case the negative) supply voltage terminal. The resistors 5 and 6 preferably have a mutually identical ohmic value.

The output winding 7 of the transformer is connected via two relay contacts 8 and 9 of the relay 10 to an output circuit comprising an indicator lamp 11, a short-circuit relay 12 having short-circuiting relay contacts 12a, a bridge rectifier 14 and the mutually coupled three-position switches 15a, 15b.

The control voltage inputs 1c, 2c of the converters 1 and 2 are connected to outputs of a waveform generator 16 which generates pulse voltages for controlling the two converters 1 and 2 on the basis of control signals originating from an amplitude adjustment circuit 17 and a current form selection circuit 18. Information on the operation and the adjustment of the waveform generator 16 may be rendered visible, if necessary, on a display unit 19.

The pulse-type control voltages delivered by the waveform generator 16 control the two voltage/current converters 1 and 2 alternately in a manner such that an alternating current of predetermined level and predetermined frequency related to the level and the frequency of the pulse-type control voltage starts to flow in the primary winding 3 of the transformer 4. As a consequence of this, a current of predetermined level (depending on the turns ratio) and a frequency corresponding to the frequency on the primary side is also induced in the output winding 7. If the switches 15a/15b are set to the lowermost position, this output current can be fed from the secondary winding 7 via the closed relay contacts 8 and 9 direct to the output terminals 39 and 40 to which the treatment electrodes may be connected. If it is desired to feed a rectified current to the output terminals 39 and 40, the switches 15a/15b are set to the position shown or to the central position so that either a positive or a negative rectified current is presented to the output terminals 39 and 40.

Most of the components in Figure 1 which have still not been discussed until now form part of a protective circuit which serves to cause a relay 10 to switch over when a predetermined current parameter is exceeded and consequently to open the contacts 8 and 9 so that the output circuit is decoupled. This protective circuit will be discussed in detail below.

As shown in Figure 1, the voltage across the resistor 5 is compared in a comparator 20 with the amplitude which is fixed by the user with a diagrammatically shown adjustment unit 17. In its simplest embodiment, this adjustment unit comprises a potentiometer whose intrinsic resistor is connected between a fixed voltage Vs and earth, while the adjustment voltage is tapped off via the slider. If the difference between the two voltages becomes larger than a predetermined limit value, the comparator 20 delivers an output signal via the conductor 21 to the relay 10, as a result of which said relay will be operated and the normally closed contacts 8 and 9 will be opened.

In a similar manner, the voltage across the resistor 6 is compared in the comparator 22 with the amplitude set with the aid of the amplitude adjustment unit 17. Here too, if the difference between the two voltages becomes larger than a predetermined limit value, the comparator 22 will deliver an output signal via the conductor 21 to the relay 10, as a result of which said relay is operated and the normally closed contacts 8 and 9 are opened.

The level of the current which has to be fed to the patient via the outputs 39 and 40 is adjusted by the patient or by the user of the apparatus with the aid of the amplitude adjustment unit 17. Depending on various factors, this current level will have to be relatively low in one case and set to a relatively high value in another case. Irrespective of the setting, however, this current level, viewed as a percentage, will not be allowed to exceed a predetermined limit value. The said abovementioned predetermined limit value may, for example, be equal to 150%. That is to say, if the voltage across one of the resistors 5 or 6 exceeds 1.5 times the amplitude which has been set with the aid of the adjustment unit 17, the relay 10 is operated and the output circuit is decoupled. Depending on the requirements which are imposed on the apparatus, another value can, of course, be chosen for this value of 150%. As will be known to a person skilled in the art, the percentage limit value may be set with the aid of suitable adjustment means for the comparators. This adjustment may either be fixed and be carried out in the factory, or may be capable of adjustment by the user within certain limits.

It is not only the voltages across the resistors 5 and 6 separately, and consequently the currents which flow through the converters 1 and 2 and consequently also through the primary winding 3 of the transformer 4 which are monitored, but also the sum of the two currents is monitored in order to prevent the total output current exceeding a predetermined limit value which must not be exceeded under any circumstances regardless of the setting of the amplitude adjustment unit. This monitoring is carried out by means of the resistors 23 and 24. These resistors have mutually equal values and are preferably of a relatively high ohmic resistance compared with the resistors 5 and 6. The resistors 23 and 24 are connected mutually in series parallel to the series circuit of the resistors 5 and 6. The junction between the resistors 23 and 24 is connected to one input of a comparator 25 whose other input receives a fixed adjustable voltage originating from a source 26. The comparator 25 compares the voltage at the junction between the resistors 23 and 24, which voltage is representative of the sum of the currents through the two converters 1 and 2, with the voltage originating from the reference source 26 and delivers an output signal to the conductor 21 if the voltage at the junction between the resistors 23 and 24 exceeds the voltage of the source 26. In that case, too, the relay 10 is thus operated and the output circuit is decoupled. It will be clear that this limit value which must not be exceeded is preferably permanently set so that neither the user nor the patient is able to exert any influence thereon.

Preferably the comparator has an adjustable frequency characteristic so that the instantaneous limit value depends on the instantaneous frequency of the current through the converters 1 and 2.

Finally, the apparatus in Figure 1 comprises a comparator 27 with which the voltage at the junction of a resistor 28 and a capacitor 29 is compared with the voltage originating from a reference voltage source 30. It will be clear that the voltage across the capacitor 29 will rise as the current pulses through the resistor 6 become wider. If the current pulses become too wide, then the comparator 27 will respond and deliver via the conductor 21 an output signal to the relay 10, as a result of which the output circuit is decoupled. This prevents the possibility that, despite a constant primary current level, a change in the secondary current level is produced because the shape of the primary current pulses changes.

If necessary, a similar detection circuit may also be connected to resistor 5, but this is not indicated in the figure.

Although mention is made in the above of "operating" the relay 10, it is preferable to incorporate the relay 10 in the circuit in a manner such that the relay is energized under normal circumstances, in which state the contacts 8 and 9 are closed. The relay is then "operated" by switching off the energizing current, as a result of which the relay is released and the contacts 8 and 9 open.

Any relay operating signal on the conductor 21 is also fed to the waveform generator 16 via the resistor 31. This responds thereto by energizing a short-circuit relay 12 so that the contacts 12a thereof are closed and the output voltage actually becomes 0 volt. As a result of this any current peaks from the transformer 7 are short-circuited if the relay 10 has still not been operated (released) and the contacts 8 and 9 have still not been opened. The relay furthermore ensures shortcircuiting of the output in the unlikely event that the contacts 8 and 9 remain stuck in the closed position for one reason or another even if the relay is operated (the relay is released).

The operation of the circuit described above is as follows. The desired current level is set by the user of the circuit or by the patient with the aid of the adjustment unit 17. A choice is made between direct current and alternating current with the aid of the switches 15a, 15b, and the pulse shape is chosen with the adjustment unit 18, that is to say, the frequency of the pulses and the duty ratio. The resultant adjustment signals of the amplitude adjustment unit 17 and of the pulse shape adjustment unit 18 are fed to the waveform generator 16 via the conductor 37, in which the resistor 38 is incorporated, or via the conductor 36. If a direct current is chosen, it is preferable that the duty ratio of the pulses generated is equal to 50% so that the ripple in the rectified current at the secondary side is at least approximately equal to zero. In order to achieve that, a coupling could be provided between the switches 15a/15b and the adjustment unit 18.

Regardless of the choice between direct current or alternating current, pulse-type control signals are generated by the waveform generator 16 for the voltage/current converters 1 and 2. If direct current has to be presented to the output terminals 39 and 40 of the circuit, which are in turn connected to the electrodes which can be placed on predetermined body parts of a patient, then although the converters 1 and 2 generate, under the control of the waveform generator 16, current pulses which are transmitted via the transformer 4 to the output circuit, these current pulses are rectified in the rectifier 14 to produce a direct current, which direct current is delivered via the switches 15a, 15b to the output terminals 39 and 40.

The polarity of the direct current to be delivered can be chosen by a correct choice of the position of the switches 15a and 15b.

If alternating current pulses have to be delivered to the output terminals 39 and 40, the switches 15a and 15b are set to the third position in which the conductors 32 and 33 are connected directly to the terminals 39 and 40.

With the aid of the indicator lamp 11 it is possible to check whether current is actually flowing through the output circuit and the load (patient) connected thereto.

If, for any reason whatsoever, the current through one of the resistors 5 and 6 (and consequently, also the current which is fed to the output terminals 39 and 40) becomes so high that the percentage limit value is exceeded, at least one of the comparators 20 or 22 will come into operation and the relay 10 will be operated. If the sum of the two currents exceeds an absolute limit value, the comparator 25 will respond and operate the relay 10. If, for any reason whatsoever, the pulse shape is altered in a manner such that this results in too high a current level, the comparator 27 will respond and energize the relay 10. In all these cases, the short-circuit relay 12 will also be operated.

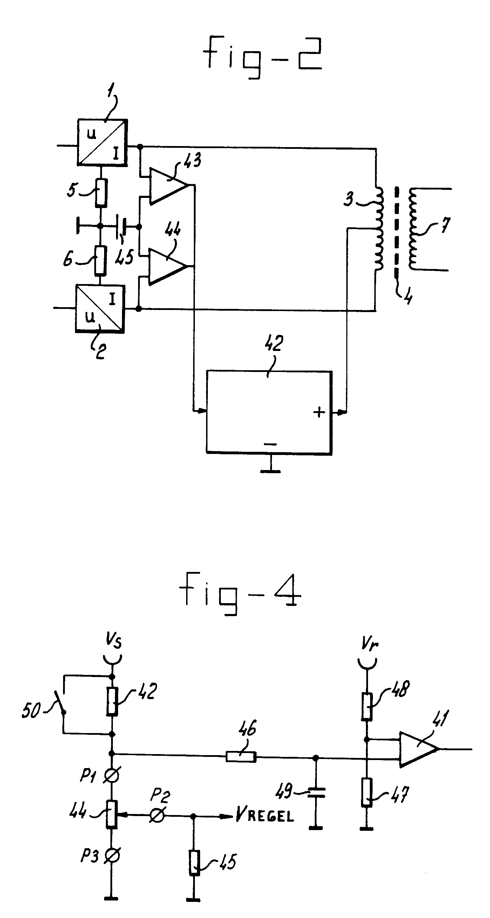

Figure 2 shows the manner in which the final stage is preferably supplied. Figure 2 shows only some of the components from Figure 1, viz. the two converters 1 and 2 and the resistors 5 and 6. Figure 2 further shows a controllable supply unit 42 whose negative terminal is in this case connected to earth and whose positive terminal is connected to a centre tapping of the primary winding 3 of the transformer 4. The control terminal of the supply unit 42 is connected to the outputs of two comparators 43 and 44. One input of the two comparators 43 and 44 is connected to one pole of a reference voltage source 45, whose other pole is earthed. The other inputs of the two comparators 43 and 44 are connected to the respective current outputs of the converters 1 and 2 in the manner shown in Figure 2.

The comparators 43 and 44 deliver a control signal to the voltage regulator 42 depending on the sum of the voltage across the resistor 5 and the reference voltage source 45, or the resistor 6 and the reference voltage source 45 respectively. If the sum of these voltages increases, a higher current therefore has to be supplied by the converter concerned and the voltage on the centre tapping of the primary winding 3 must preferably become higher in order to compensate for the voltage loss of the resistor 5 or 6 and any internal voltage losses in the converters. The output voltage of the regulator 42, which is fed to the centre tapping of the winding 3, therefore increases as more current is demanded. This achieves the result that the dissipation in the two converters 1 and 2 can be kept relatively low and virtually constant.

The transformer 4 is preferably so constructed that this transformer becomes saturated at a predetermined current level, as a result of which a further increase in current is limited and an additional protection is consequently created.

Figure 3 shows another embodiment of the apparatus according to the invention. Components which are also used in the embodiment according to Figure 1 are provided with the same reference symbols. The most important difference between the embodiments of Figures 1 and 3 is to be found in the circuit around the adjustment unit 17. In Figure 3, the adjustment unit 17 is provided with a potentiometer 44 which is connected in series with a fixed resistor 42 between a fixed voltage terminal Vs and earth. The junction between the resistors 42 and the potentiometer 44 is connected to one input of a further comparator 41 whose other input is connected to a reference voltage source 43.

With the aid of the comparator 41, a check is made on whether the voltage at the junction between the resistor 42 and the potentiometer 44 has a value which is lower than the fixed voltage Vs by at least a predetermined value. In the normal case this has to be so. However, should the connection between earth and the potentiometer 44 become detached for any reason whatsoever, the voltage on the slider of the potentiometer 44, and consequently the control voltage on one input of the comparators 20 and 22, would increase considerably in an undesirable manner as a result without the protection responding. The reason is that the comparators 20 and 22 only respond if the current through the converters 1 and 2 exceeds 150% (or any other set value) of the set current. However, if the adjustment current itself changes, the comparators 20 and 22 do not respond thereto. This problem is now eliminated by adding the resistor 42 in combination with the comparator 41. In the normal case the voltage at the junction between the resistor 42 and the potentiometer 44 will never exceed a certain value which is related to the dimensioning of the resistors 42 and 44, the voltage Vs and the impedance which loads the slider of the potentiometer 44. However, if the connection between the potentiometer 44 and earth becomes detached, the potential at the junction between the resistor 42 and the potentiometer 44 will increase in a manner such that the comparator 41 detects an increase above the set limit value. As a result thereof, the comparator 41 delivers a signal which operates the relay 10 so that the contacts 8 and 9 are opened.

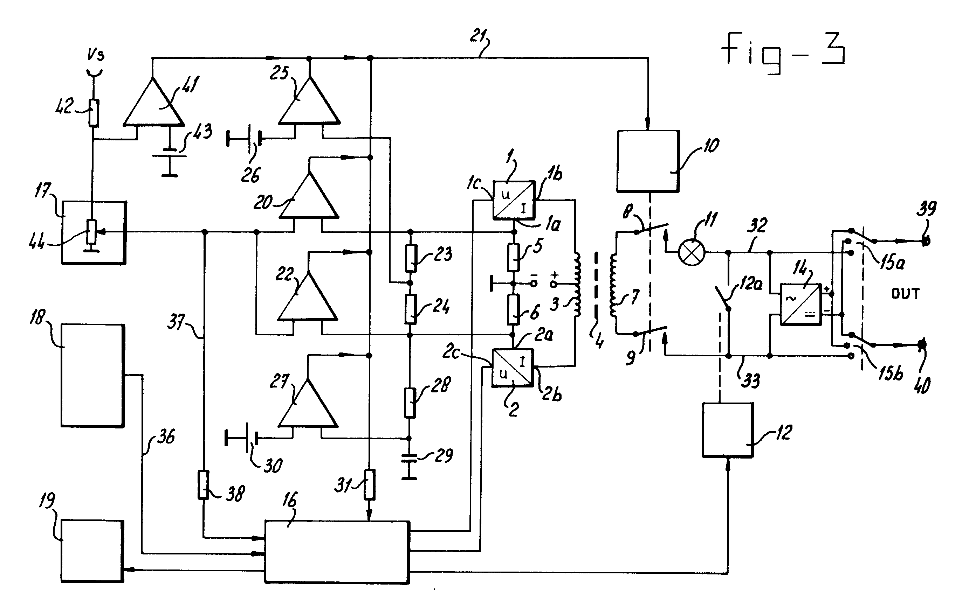

Figure 4 shows a more detailed and further developed embodiment of the circuit around the potentiometer 44 in the amplitude adjustment unit 17.

The circuit in Figure 4 again comprises the potentiometer 44 having the terminal contacts P1, P2 and P3, and the resistor 42 which is connected in series with the potentiometer 44 between the fixed voltage terminal Vs and earth. Furthermore, the slider of the resistor 44 is loaded by a slider loading resistor 45. The voltage at the junction between the resistor 42 and the potentiometer 44 is fed via a resistor 46 to one input of the comparator 41. The reference voltage, which has to be fed to the other input of the comparator 41, is obtained via a voltage divider comprising the resistors 47 and 48 fitted between earth and a fixed reference potential Vr. Finally, the said first input of the comparator 41 is furthermore decoupled via a capacitor 49. A switch 50 is furthermore fitted in parallel with the resistor 42 for test purposes.

The circuit from Figure 4 provides a high degree of protection even in the unlikely event of one of the terminals P1, P2 or P3 of the potentiometer becoming detached or of any other defects occurring in the circuit, as is evident from the following analysis.

If the connection at the position of the potentiometer terminal P1 should become detached, the regulating voltage Vr which is fed both to the waveform generator 16 and to the comparators 20 and 22 becomes equal to zero as a result thereof. The sole result thereof is that the output current also drops to zero.

If the slider terminal becomes detached, the regulating voltage V_{regel} also becomes zero as a result of the presence of the slider load resistor 45. In this case, too, no dangerous situation arises for the patient.

If the earth terminal P3 of the potentiometer should become detached, an excessive increase in voltage at the junction between the resistors 42 and 44 is produced, as already explained with reference to Figure 3, which is sufficient to switch the comparator to the other setting so that a signal is delivered to the protective relay 10.

The test switch 50 can be used to check the satisfactory operation of the comparator 41. If the switch 50 is depressed, the voltage at the junction between the resistor 42 and the potentiometer 44 will increase as a result thereof to the potential of the fixed voltage terminal Vs. The comparator 41 of necessity responds thereto by operating the protective relay and opening the contacts 8 and 9, and this can be checked in a simple manner.

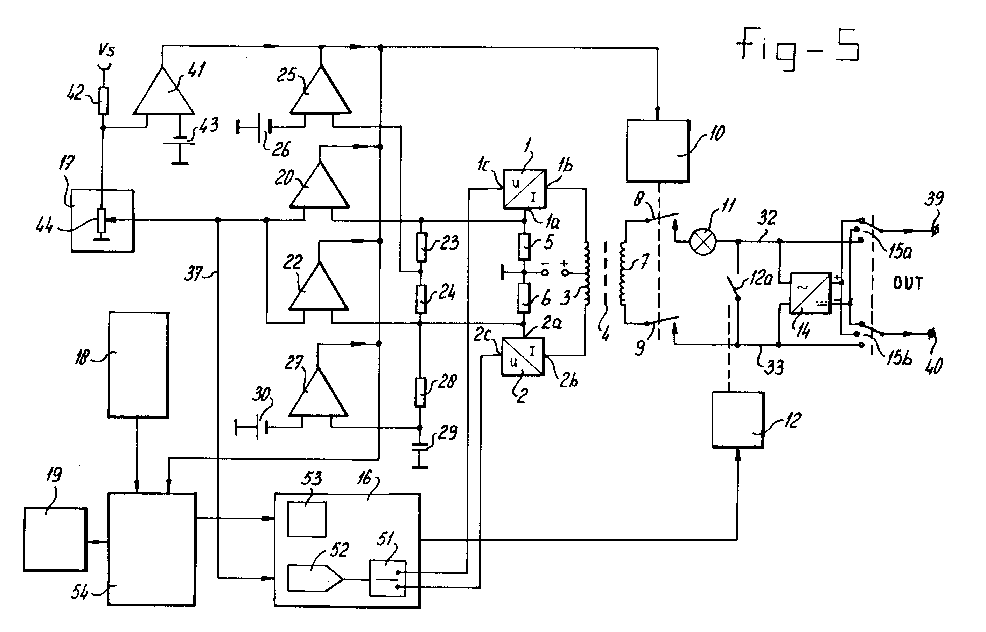

Finally, Figure 5 shows yet another, further developed embodiment of the apparatus according to the invention. The difference between the embodiments of Figures 3 and 5 is that in Figure 5 a microprocessor is used which, on the one hand, delivers control signals to the waveform generator 16, but is essentially used for test purposes, as will be further discussed briefly below. The components in Figure 5 which correspond to components from Figure 3 are indicated by the same reference numerals.

The waveform generator 16 is provided internally with a chopper 51, a digital/analogue converter 52 and a waveform table memory 53. The amplitude adjustment signal originating from the adjustment unit 17 is converted by the converter 52 into a signal of predetermined level intended for controlling the converters 1 and 2. This signal is alternately presented via the chopper 51 to the converters 1 and 2. The desired waveform of the output signal is set by means of a correct choice of one of the waveforms stored in the memory 53. This choice is made by means of the adjustment unit 18 which generates via the microprocessor 54 a control signal for the waveform generator 16. Since the detailed operation of the waveform generator 16 is not relevant in relation to the invention, a more detailed description of the waveform generator 16 is considered superfluous. However, in relation to a detailed elaboration of this generator, attention is drawn to the literature from which diverse waveform generators constructed with the aid of digital/analogue converters and choppers are known.

As has already been pointed out, the microprocessor 54 has the important task of testing the entire apparatus. A possible test procedure will be briefly discussed below.

As soon as the supply voltage has reached its stable final value, the self-testing of the apparatus starts. After the appearance of the supply voltage, the apparatus is in a safe initial stage. The user of the apparatus must not operate any of the adjustment devices of the apparatus during the self-testing and, moreover, the adjustment unit must be in the zero position. The microprocessor 54 checks this regularly and regards any deviations from these requirements as a fault, the response to which is as will be further described below.

First of all, under the control of a suitable program stored in the microprocessor 54 for this purpose, the microprocessor 54 carries out the following operations in succession:
1. A check is made on whether the protective relay is switched off.
2. The registers of the microprocessor are checked internally. For this purpose, a register is filled with a particular bit pattern, said bit pattern is read out of the register again and then compared with the original bit pattern.
3. The arithmetic unit of the microprocessor is checked by carrying out a number of arithmetical instructions and comparing the calculated values with fixed correct values.
4. The program memory, and consequently also the address/databus of the processor, are checked by carrying out a check sum on the program memory and comparing the latter with a stored correct value.
5. The data memory of the microprocessor is checked by writing a predetermined bit pattern into the memory, reading out said bit pattern again and comparing it with the original bit pattern.
6. The time-determining units in the microprocessor are checked by allowing each unit to measure a predetermined period and comparing this period with the result generated with the aid of a delay loop which delays by the same period.
7. The microprocessor 16 is provided with an analogue/digital converter which digitizes, in particular, the set value from the amplitude adjustment unit 17. A reference voltage is fed to this A/D converter as a test and the digitized result is compared with an expected correct value.
8. The voltages at various points in the apparatus are measured by means of the A/D converter which has now been checked and checked with correct stored values.

If a divergence is detected anywhere in this test procedure, or if an operating fault is detected, the test cycle stops and a fault message is delivered via the indicating unit 19. The apparatus cannot be operated further and has to be switched off completely before a new attempt can be undertaken.

If the operation of the microprocessor 54 is found to be in order, the operation of the protective relay 10 and of the short-circuit relay 12 is then tested. In this case, a patient must not be connected. The test is carried out by presenting a known voltage to the input of the A/D converter (corresponding to a predetermined setting of the adjustment unit 17) as a result of which a predetermined current will start to flow through the output circuit if the output circuit is not open-circuit (the contacts 8 and 9 are closed). The A/D converter is then set to a level which corresponds to a value higher than the maximum permissible output current. The result thereof must be that the protective relay switches. The reason is that on switching on the apparatus the amplitude adjustment unit 17 must be at zero so that the protective relay 10 must come into operation directly on carrying out this test.

The individual comparators which are incorporated in the protective circuit can be tested in a similar manner.

## Claims

1. Electrotherapy apparatus which is capable of generating direct currents or alternating currents which are intended to be applied to a patient via electrodes, which apparatus comprises:
- two voltage/current converters (1, 2), each provided with a current input (1a, 2a), a current output (1b, 2b) and a control voltage input (1c, 2c), the current input of each of these converters (1, 2) being connected via a resistor (5, 6) to a first supply voltage terminal and the control voltage input being connected to a waveform generator (16),
- a transformer (4) having a primary (3) and a secondary winding (7), which secondary winding is connected via at least one relay contact (8, 9) to an output circuit which can be connected to the said electrodes, the two ends of the primary winding (3) each being connected to a current output (1b, 2b) of one of the said converters (1, 2) and a centre tapping of the primary winding being connected to the second supply voltage terminal,
- there being connected to the two said resistors (5, 6) a protective circuit which monitors the current through said resistors during operation and which, if a predetermined current is exceeded, operates a relay (10), as a result of which the said at least one relay contact (8, 9) is switched over and the connection between the output circuit and the secondary winding (7) of the transformer (4) is interrupted.

2. Electrotherapy apparatus according to claim 1, characterized in that the protective circuit is provided with a first and a second comparator (20, 22), each having an input connected to the junction between one of the said resistors (5, 6) and the current input (1a, 2a) connected thereto, and each having the other input connected, or connectable, to a reference voltage source (17) in a manner such that, during operation, the voltage across each of the said resistors (5, 6) is compared in a comparator (20, 22) with a predetermined reference voltage and that, if the reference voltage is exceeded, the comparator (20, 22) concerned generates an energizing signal for the said relay (10).

3. Electrotherapy apparatus according to claim 2, characterized in that the apparatus is provided with adjustment means with which the reference voltage can be set by the user of the apparatus.

4. Electrotherapy apparatus according to claim 3, characterized in that the adjustment means are provided with a potentiometer (44), the resistor body being connected, on the one hand to earth and being connected, on the other hand, via a resistor (42) to a fixed voltage terminal (Vs), the slider of the potentiometer being connected to the said comparators (20, 22) and the junction between the potentiometer (44) and the said resistor (42) being connected to one input of a further comparator (41), the other input of which is connected to a predetermined fixed potential (43) and, if the potential at the junction between the potentiometer and the resistor rises above a predetermined threshold value, the further comparator (41) generates an operating signal for the said relay (10).

5. Electrotherapy apparatus according to claim 4, characterized in that a slider loading resistor (45) is fitted between slider and earth.

6. Electrotherapy apparatus according to claim 3, 4, or 5, characterized in that the adjustment means are so constructed that the reference voltage can be varied individually for each comparator.

7. Electrotherapy apparatus according to one of the preceding claims, characterized in that the protective circuit is provided with a third comparator (25), one input of which is connected via a resistor network (23, 24) to the said resistors (5, 6) and the other input of which is connected, or is connectable, to a limit voltage source (26) in a manner such that, during operation, the sum of the voltages across the said resistors (5, 6) is compared in the third comparator (25) with a predetermined limit value, the third comparator generating an operating signal for the said relay (10) if the limit value is exceeded.

8. Electrotherapy apparatus according to claim 7, characterized in that the limit voltage source is provided with a frequency dependent network such that the instantaneous limit value is dependent on the instantaneous frequency of the sum of the currents through the said resistors.

9. Electrotherapy apparatus according to one of the preceding claims, characterized in that the protective circuit is provided with a fourth comparator (27), one input of which is connected via an integrating network (28, 29) to the junction between one of the said resistors (5,6) and the associated current input and the other input of which is connected to a standard voltage source (30) in a manner such that, during operation, the integrated voltage across the said one resistor concerned is compared with the standard voltage and that, if the standard voltage is exceeded, an operating signal is generated by the fourth comparator (27) for the said relay (10).

10. Electrotherapy apparatus according to claim 9, characterized in that the protective circuit is provided with a fifth comparator, one input of which is connected via an integrating network to the cross-over point between the other said resistor and the associated current input and the other input of which is connected to a standard voltage source in a manner such that, during operation, the integrated voltage across the other said resistor concerned is compared with the standard voltage and that, if the standard voltage is exceeded, an operating signal is generated by the fifth comparator for the said relay.

11. Electrotherapy apparatus according to one of the preceding claims, characterized in that the waveform generator (16) is provided with an input to which a signal can be presented, in response to which the waveform generator drives the two voltage current converters to a state in which no current is delivered to the current output, which input is driven, during operation, by the operating signal of the protective circuit.

12. Electrotherapy apparatus according to one of the preceding claims, characterized in that the transformer is so constructed that it becomes saturated at a predetermined current level related to the safety requirements.

## Patentansprüche

1. Elektrotherapiegerät zur Erzeugung von Gleich- bzw. Wechselströmen, die über Elektroden einem Patienten zugeführt werden sollen, das umfaßt:
zwei Strom/Spannungs-Wandler (1, 2), von denen jeder einen Stromeingang (1a, 2a), einen Stromausgang (1b, 2b) und einen Steuerspannungseingang (1c, 2c) hat, wobei der Stromeingang von jedem Wandler (1, 2) über einen Widerstand (5, 6) mit einem ersten Spannungsversorgungsstecker verbunden ist und der Steuerspannungseingang mit einem Wellenformgenerator (16) verbunden ist,
einen Transformator (4) mit einer Primär- (3) und einer Sekundärwicklung (7), wobei die Sekundärwicklung über wenigstens einen Relaiskontakt (8, 9) mit einem Ausgangsschaltkreis verbunden ist, welcher mit den Elektroden verbunden sein kann, und die zwei Enden der Primärwicklung (3) jeweils mit dem Stromausgang (1b, 2b) eines der Wandler (1, 2) verbunden sind und ein Zentralabgriff der Primärwicklung mit der Anschlußklemme für die zweite Spannungsversorgung verbunden ist,
einen Schutzschaltkreis, der mit zwei Widerständen (5, 6) verbunden ist und welcher den Strom durch diese Widerstände bei Betrieb überwacht und bei Überschreiten eines vorgegebenen Stromwertes ein Relais (10) schaltet, wodurch wenigstens ein Relaiskontakt (8, 9) umgeschaltet wird und die Verbindung zwischen dem Ausgangsschaltkreis und der Sekundärwicklung (7) des Transformators (4) unterbrochen wird.

2. Elektrotherapiegerät nach Anspruch 1,
dadurch gekennzeichnet,
daß der Schutzschaltkreis mit einem ersten und einem zweiten Vergleicher (20, 22) versehen ist, von denen jeder einen Eingang hat, der mit der Verbindung zwischen einem der Widerstände (5, 6) und dem damit zusammengeschlossenen Stromeingang (1a, 2a) verbunden ist, und von denen der jeweils andere Eingang verbunden ist oder verbunden werden kann mit einer Referenzspannungsquelle (17), so daß bei Betrieb die über jeden Widerstand (5, 6) abfallende Spannung in, dem Vergleicher (20, 22) mit einer vorgegebenen Referenzspannung verglichen wird und bei Überschreiten der Referenzspannung durch den entsprechenden Vergleicher (20, 22) ein Umschaltsignal für das Relais (10) ausgegeben wird.

3. Elektrotherapiegerät nach Anspruch 2,
dadurch gekennzeichnet,
daß das Gerät eine Einstellvorrichtung hat, mit welcher die Referenzspannung durch den Benutzer des Gerätes eingestellt werden kann.

4. Elektrotherapiegerät nach Anspruch 3,
dadurch gekennzeichnet,
daß die Einstellvorrichtung mit einem Potentiometer (44) ausgestattet ist, der Widerstandskörper auf einer Seite mit Masse verbunden ist und auf der anderen Seite über einen Widerstand (42) mit einer Anschlußklemme, die auf fester Spannung (Vs) liegt, der Schleifer des Potentiometers mit den Vergleichern (20, 22) verbunden ist und die Verbindung zwischen dem Potentiometer (44) und dem Widerstand (42) mit dem Eingang eines weiteren Vergleichers (41) verbunden ist, dessen anderer Eingang auf vorgegebenem festen Potential (43) liegt, und daß bei Überschreiten des Potentials an der Verbindungsstelle zwischen Potentiometer und Widerstand eines bestimmten Durchschaltwertes durch den weiteren Vergleicher (41) ein Schaltsignal für das Relais (10) ausgegeben wird.

5. Elektrotherapiegerät nach Anspruch 4,
dadurch gekennzeichnet,
daß zwischen Schleifer und Masse ein Schleifer-Lastwiderstand (45) liegt.

6. Elektrotherapiegerät nach einem der Ansprüche 3 bis 5,
dadurch gekennzeichnet,
daß die Einstellungsvorrichtung so aufgebaut ist, daß die Referenzspannung unabhängig für jeden Vergleicher eingestellt werden kann.

7. Elektrotherapiegerät nach einem der vorangehenden Ansprüche,
dadurch gekennzeichnet,
daß der Schutzschaltkreis mit einem dritten Vergleicher (25) versehen ist, von dem ein Eingang über ein Widerstandsnetzwerk (23, 24) mit den Widerständen (5, 6) verbunden ist und dessen anderer Eingang verbunden ist bzw. verbunden werden kann mit einer Grenzspannungsquelle (26), so daß bei Betrieb die Summe der Spannungen über die Widerstände (5, 6) durch einen dritten Vergleicher (25) mit einem vorgegebenen Grenzwert verglichen wird, wobei der dritte Vergleicher ein Schaltsignal für das Relais (10) ausgibt, wenn der Grenzwert überschritten wird.

8. Elektrotherapiegerät nach Anspruch 7,
dadurch gekennzeichnet,
daß die Grenzspannungsquelle mit einem frequenzabhängigen Netzwerk versehen ist, so daß der augenblickliche Grenzwert von der augenblicklichen Frequenz der Summe der Ströme durch die Widerstände abhängt.

9. Elektrotherapiegerät nach einem der vorangehenden Ansprüche,
dadurch gekennzeichnet,
daß der Schutzschaltkreis mit einem vierten Vergleicher (27) versehen ist, dessen einer Eingang über ein integrierendes Netzwerk (28, 29) mit der Verbindung zwischen einem der Widerstände (5, 6) und dem dazugehörigen Stromeingang verbunden ist, und dessen anderer Eingang mit der Standardspannungsquelle (30) verbunden ist, so daß bei Betrieb die integrierte Spannung über den einen betreffenden Widerstand mit der Standardspannung verglichen wird und bei Überschreiten dieser Standardspannung ein Schaltsignal durch den vierten Vergleicher (27) für das Relais (10) ausgegeben wird.

10. Elektrotherapiegerät nach Anspruch 9,
dadurch gekennzeichnet,
daß der Schutzschaltkreis mit einem fünften Vergleicher versehen ist, dessen einer Eingang über ein integrierendes Netzwerk mit dem Überkreuzungs-Punkt zwischen dem anderen besagten Widerstand und dem dazugehörigen Stromeingang verbunden ist, und dessen anderer Eingang mit einer Standardspannungsquelle verbunden ist, so daß bei Betrieb die integrierte Spannung über den anderen betreffenden Widerstand mit der Standardspannung verglichen wird und bei Überschreiten der Standardspannung ein Schaltsignal durch den fünften Vergleicher für das Relais ausgegeben wird.

11. Elektrotherapiegerät entsprechend einem der vorangehenden Ansprüche,
dadurch gekennzeichnet,
daß der Wellenformgenerator (16) mit einem Eingang versehen ist, an dem ein Signal anliegen kann, auf das hin der Wellenformgenerator die beiden Strom/Spannungswandler ansteuert und in einen Schaltzustand bringt, in welchem kein Strom zum Ausgang fließt, wobei an dem Eingang bei Betrieb das Schaltsignal des Schutzschaltkreises anliegt.

12. Elektrotherapiegerät nach einem der vorangehenden Ansprüche,
dadurch gekennzeichnet,
daß der Transformator so aufgebaut ist, daß er sättigt bei einem bestimmten Strom, der von den Sicherheitsanforderungen abhängt.

## Revendications

1. Appareil d'électrothérapie qui est capable de générer des courants continus ou des courants alternatifs qui sont prévus pour être appliqués sur un patient au moyen d'électrodes, lequel appareil comporte :
- deux convertisseurs tension/courant (1, 2) pourvus chacun d'une entrée de courant (1a, 2a), d'une sortie de courant (1b, 2b) et d'une entrée de tension de commande (1c, 2c), l'entrée de courant de chacun de ces convertisseurs (1, 2) étant reliée par l'intermédiaire d'une résistance (5, 6) à une première borne de tension d'alimentation et l'entrée de tension de commande étant reliée à un générateur d'onde (16),
- un transformateur (4) ayant un enroulement primaire (3) et un enroulement secondaire (7), lequel enroulement secondaire est relié à l'aide d'au moins un contact de relais (8, 9) à un circuit de sortie qui peut être relié auxdites électrodes, les deux extrémités de l'enroulement primaire (3) étant chacune reliée à une sortie de courant (1b, 2b) de l'un desdits convertisseurs (1, 2) et un piquage central de l'enroulement primaire étant relié à la deuxième borne de tension d'alimentation,
- un circuit de protection qui est relié auxdites deux résistances (5, 6) qui contrôle le courant à travers lesdites résistances pendant le fonctionnement et qui, si un courant prédéterminé est dépassé, actionne un relais (10), avec pour résultat qu'au moins un contact dudit relais (8, 9) est basculé et la liaison entre le circuit de sortie et l'enroulement secondaire (7) du transformateur (4) est interrompue.

2. Appareil d'électrothérapie selon la revendication 1, caractérisé en ce que le circuit de protection est pourvu d'un premier et d'un deuxième comparateur (20, 22), ayant chacun une entrée reliée à la jonction entre l'une desdites résistances (5, 6) et l'entrée de courant (1a, 2a) qui y est reliée, et ayant chacun l'autre entrée reliée, ou pouvant être reliée, à une source de tension de référence (17) d'une manière telle que, pendant le fonctionnement, la tension aux bornes de chacune desdites résistances (5, 6) est comparée dans un comparateur (20, 22) à une tension de référence prédéterminée et que, si la tension de référence est dépassée, le comparateur (20, 22) concerné génère un signal d'excitation pour ledit relais (10).

3. Appareil d'électrothérapie selon la revendication 2, caractérisé en ce que l'appareil est pourvu de moyens de réglage avec lesquels la tension de référence peut être réglée par l'utilisateur de l'appareil.

4. Appareil d'électrothérapie selon la revendication 3, caractérisé en ce que les moyens de réglage sont pourvus d'un potentiomètre (44), le corps de résistance étant relié d'une part à la masse et étant relié, d'autre part, par l'intermédiaire d'une résistance (42) à une borne de tension fixe (Vs), le curseur du potentiomètre étant relié auxdits comparateurs (20, 22) et la jonction entre le potentiomètre (44) et ladite résistance (42) étant reliée à une entrée d'un autre comparateur (41), dont l'autre entrée est reliée à un potentiel fixe prédéterminé (43) et, si le potentiel à la jonction entre le potentiomètre et la résistance passe au-dessus d'une valeur de seuil prédéterminée, l'autre comparateur (41) génère un signal d'actionnement pour ledit relais (10).

5. Appareil d'électrothérapie selon la revendication 4, caractérisé en ce qu'une résistance de charge de curseur (45) est prévue entre le curseur et la masse.

6. Appareil d'électrothérapie selon la revendication 3, 4 ou 5, caractérisé en ce que les moyens de réglage sont construits de telle sorte que la tension de référence peut être modifiée individuellement pour chaque comparateur.

7. Appareil d'électrothérapie selon l'une des revendications précédentes, caractérisé en ce que le circuit de protection est pourvu d'un troisième comparateur (25) dont une entrée est reliée par l'intermédiaire d'un réseau de résistance (23, 24) aux dites résistances (5, 6) et dont l'autre entrée est reliée, ou peut être reliée, à une source de tension limite (26) d'une manière telle que, pendant le fonctionnement, la somme des tensions aux bornes desdites résistances (5, 6) est comparée dans le troisième comparateur (25) à une valeur limite prédéterminée, le troisième comparateur générant un signal d'actionnement pour ledit relais (10) si la valeur limite est dépassée.

8. Appareil d'électrothérapie selon la revendication 7, caractérisé en ce que la source de tension limite est pourvue d'un réseau dépendant de la fréquence de telle sorte que la valeur limite instantanée dépend de la fréquence instantanée de la somme des courants aux bornes desdites résistances.

9. Appareil d'électrothérapie selon l'une des revendications précédentes, caractérisé en ce que le circuit de protection est pourvu d'un quatrième comparateur (27), dont une entrée est reliée par l'intermédiaire d'un réseau d'intégration (28, 29) à la jonction entre l'une desdites résistances (5, 6) et l'entrée de courant associée et dont l'autre entrée est reliée à une source de tension standard (30) d'une manière telle que, pendant le fonctionnement, la tension intégrée aux bornes de ladite résistance concernée est comparée à la tension standard et que, si la tension standard est dépassée, un signal d'actionnement est généré par le quatrième comparateur (27) pour ledit relais (10).

10. Appareil d'électrothérapie selon la revendication 9, caractérisé en ce que le circuit de protection est pourvu d'un cinquième comparateur, dont une entrée est reliée par l'intermédiaire d'un réseau d'intégration au point de raccordement entre l'autre résistance et l'entrée de courant associé et dont l'autre entrée est reliée à une source de tension standard d'une manière telle que, pendant le fonctionnement, la tension intégrée aux bornes de l'autre dite résistance concernée est comparée à la tension standard et que si la tension standard est dépassée, un signal d'actionnement est généré par le cinquième comparateur pour ledit relais.

11. Appareil d'électrothérapie selon l'une des revendications précédentes, caractérisé en ce que le générateur d'onde (16) est pourvu d'une entrée sur laquelle peut être présenté un signal, en réponse auquel le générateur d'onde attaque les deux convertisseurs tension/courant dans un état dans lequel aucun courant n'est délivré sur la sortie de courant, laquelle entrée est attaquée pendant le fonctionnement par le signal d'actionnement du circuit de protection.

12. Appareil d'électrothérapie selon l'une des revendications précédentes, caractérisé en ce que le transformateur est construit de telle sorte qu'il devient saturé à un niveau de courant prédéterminé lié aux exigences de sécurité.
